# NEUE EUROPÄISCHE PATENTSCHRIFT

(11) **EP 0 662 476 B2**
(45) Veröffentlichungstag und Bekanntmachung des Hinweises auf die Entscheidung über den Einspruch: **14.08.2002**
(45) Hinweis auf die Patenterteilung: 20.01.1999
(21) Anmeldenummer: 94117768.5
(22) Anmeldetag: 10.11.1994
(51) Int. Cl.: C07D 487/04

(54) **Verfahren zur Herstellung Bicyclischer Amidine**
Process for the preparation of bicyclic amidines
Procédé pour la préparation d' amidines bicycliques

(30) Priorität: 11.11.1993 CH 338593
(43) Veröffentlichungstag der Anmeldung: 12.07.1995
(60) Teilanmeldung: 98110227.0
(73) Patentinhaber: LONZA AG, CH-3945 Gampel/Wallis (CH)
(72) Erfinder: Werbitzky, Oleg, Dr., CH-3930 Visp, (Kanton Wallis) (CH); Daum, Ulrich, Dr., CH-4114 Hofstetten, (Kanton Solothurn) (CH); Bregy, Rachel, CH-3942 Raron, (Kanton Wallis) (CH)
(74) Vertreter: Weinhold, Peter, Dr.rer.nat. Dipl.-Chem.

(56) Entgegenhaltungen:
- EP-A- 0 199 483
- DE-A- 1 545 855
- GB-A- 1 182 014
- PHARMAZIE, Bd.47, Nr.6, 1992 Seiten 403 - 409 MOEHRLE,H;OTTERSBACH,D; STEIGEL,A 'Ring-chain isomerism of fused alpha-amino nitrones'
- J.W. Copenhaver et al. "Acetylene and Carbon Monoxide Chemistry", Reinhold Publishing Corp., New York, 1949, Seiten 164-165

## Beschreibung

Die vorliegende Erfindung betrifft ein neues Verfahren zur Herstellung von bicyclischen Amidinen durch Umsetzung von Lactonen mit primären Aminen.

Bicyclische Amidine sind starke organisc Basen, die wegen ihrer hohen Basizität bei geringer Nucleophilie und ihrer guten Löslichkeit in fast allen Lösungsmitteln zahlreiche Anwendungen gefunden haben. Besonders bekannt sind die üblicherweise mit den Abkürzungen DBN und DBU bezeichneten Verbindungen 1,5-Diazabicyclo[4.30]non-5-en (2,3,4,6,7,8-Hexahydropyrrolo[1,2-a]pylimidin) und 1,1,8-Diazabicyclo[5.4.0]undec-7-en (2,3,4,6,7,8,9,1 O-Octahydropyrimido[1,2-a]azepin).

Eine Übersicht über Verwendungen dieser Verbindungen in chemischen Synthesen findet sich z. B. in "Synthetica Merck", Band II, E. Merck, Darmstadt, 1974, S. 118 - 119, 124.

Ein bekanntes Herstellungsverfahren für bicyclische Amidine geht von N-(ω-Aminoalkyl)lactonen aus, die beim Erhitzen mit sauren Katalysatoren unter Wasserabspaltung zu den Amidinen cyclisieren (DE-C-15 45 855).

Die N-(ω-Aminoalkyl)lactone werden beispielsweise aus den entsprechenden Cyanoverbindungen durch Hydrierung erhalten, so insbesondere das N-(γ-Aminopropyl)pyrrolidon aus dem N-(β-Cyanethyl)pyrrolidon (siehe z. B. W. Reppe et al., Justus Liebigs Ann. Chem. 1955, 596, S. 211).

Es ist auch möglich, N-(ω-Aminoalkyl)lactone aus den entsprechenden Lactonen und α,ω-Diaminoalkanen herzustellen (DE-C-730 182).

Die bekannten Verfahren zur Herstellung bicyclischer Amidine haben den Nachteil, dass sie mindestens zwei Synthesestufen mit Aufarbeitung der Zwischenprodukte umfassen.

Es ist bekannt, dass sich bicyclische Amidine gut als Katalysatoren für die Herstellung von Polyurethanen eignen (FR-A 1 542 058).

Aufgabe der vorliegenden Erfindung war daher, ein verbessertes und vereinfachtes Verfahren zur Herstellung bicyclischer Amidine aufzuzeigen. Erfindungsgemäss wird die Aufgabe durch das Herstellungsverfahren nach Patentanspruch 1 gelöst.

Es wurde gefunden, dass es möglich ist, bicyclische Amidine der allgemeinen Formel 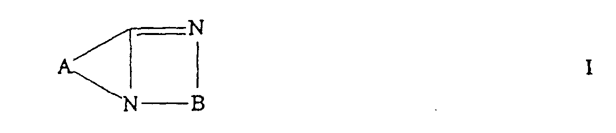 in einem Eintopfverfahren ohne Isolierung oder Reinigung von Zwischenstufen aus den entsprechenden Lactonen der allgemeinen Formel 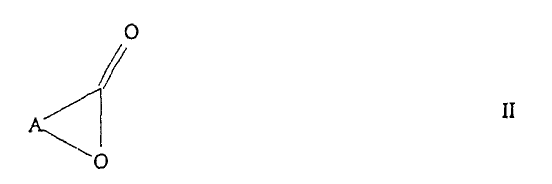 und Aminen der allgemeinen Formel

H₂N―B―NH₂ III

herzustellen.

Die Gruppe A im Lacton (II) und dem Amidin (I) steht jeweils für eine 3-, 4- oder 5-gliedrige Kohlenstoffkette der Formel -CR¹R²-CR³R⁴-CR⁵R⁶-, -CR¹R²-CR³R⁴-CR⁵R⁶-CR⁷R⁸- oder -CR¹R²-CR³R⁴-CR⁵R⁶-CR⁷R⁸-CR⁹R¹⁰-, wobei R¹ und R² jeweils an das dem Heteroatom benachbarte Kohlenstoffatom gebunden sind.

Die Gruppe B im Amin (III) und dem Amidin (I) steht jeweils für eine 2-, 3- oder 4-gliedrige Kohlenstoffkette der Formel -CR¹¹R¹²-CR¹³R¹⁴-, -CR¹¹R¹²-CR¹⁵R¹⁶-CR¹³R¹⁴- oder -CR¹¹R¹²-CR¹⁵R¹⁶-CR¹⁷R¹⁸-CR¹³R¹⁴-.

Damit umfasst die allgemeine Formel I bicyclische Amidine mit 5-, 6- oder 7-gliedrigen Ringen, wobei die beiden Ringe gleiche oder verschiedene Gliederzahl aufweisen können. Entsprechend umfasst die allgemeine Formel II Lactone mit 5 bis 7 Ringgliedern, also γ-, δ- und ε-Lactone.

Die Substituenten R¹, R² und R¹¹ bis R¹⁴ der Kohlenstoffketten A und B sind jeweils unabhängig voneinander Wasserstoff, C₁-C₄-Alkyl oder Aryl oder C₁-C₄-Alkylgruppen, welche ihrerseits mit Hydroxy, Amino, C₁-C₄-Alkylamino oder Mercapto substituiert sind. Die Substituenten R³ bis R¹⁰ und R¹⁵ bis R¹⁸ können ausser den für R¹, R² und R¹¹ bis R¹⁴ genannten Gruppen noch Hydroxy-, Amino-, C₁-C₄-Alkylamino- oder Mercaptogruppen sein.

Unter C₁-C₄-Alkyl sind hier alle primären, sekundären und tertiären unverzweigten oder verzweigten Alkylgruppen mit bis zu 4 Kohlenstoffatomen zu verstehen, also Methyl, Ethyl, Propyl, Butyl, Isopropyl, Isobutyl, sec-Butyl und tert-Butyl. Unter Aryl ist insbesondere Phenyl oder alkylsubstituiertes Phenyl zu verstehen, wie beispielsweise o-, m- oder p-Tolyl oder die verschiedenen isomeren Xylylgruppen. Als Lactone eignen sich beispielsweise γ-Butyrolacton, γ- und δ-Valerolacton, ε-Caprolacton oder substituierte Lactone wie Pantolacton (2-Hydroxy-3,3-dimethyl-γ-butyro-lacton). Funktionelle Gruppen als Substituenten, also Hydroxy, Amino, Alkylamino oder Mercapto, befinden sich vorzugsweise an der Gruppe B der Amin komponente (III).
Als Amine (III) kommen daher nicht nur primäre Diamine, sondern auch Verbindungen mit zusätzlichen primären oder sekundären Aminogruppen in Frage. Geeignete Amine sind beispielsweise 1,2-Diaminoethan, 1,2-Diaminopropan, 1,3-Diaminopropan, 1,2,3-Triaminopropan, 1,1,1-Tris(aminomethyl)ethan oder Tetrakis(aminomethyl)methan. Werden Amine mit nicht-äquivalenten primären Aminogruppen eingesetzt, wie beispielsweise 1,2-Diaminopropan oder 1,2,3-Triaminopropan, so können unter Umständen Produktgemische entstehen.

Bei der Umsetzung des Lactons (II) mit dem Amin (III) werden 2 bis 20 mol Amin auf 1 mol Lacton eingesetzt. Der Überschuss an Amin kann bei der Aufarbeitung des Reaktionsgemischs zurückgewonnen werden.

Die Reaktion wird zweckmässig bei einer Temperatur von mindestens 150°C durchgeführt. Vorzugsweise liegt die Reaktionstemperatur zwischen 200 und 300°C. Ein inertes Lösungsmittel wie beispielsweise Toluol oder Xylol kann zugesetzt werden, ist aber nicht erforderlich. Vorzugsweise wird die Reaktion ohne Lösungsmittel durchgeführt. Zur Erreichung der Reaktionstemperatur ist es im allgemeinen erforderlich, das Reaktionsgemisch unter erhöhtem Druck zu halten, weil die Siedepunkte vieler Edukte bei Normaldruck niedriger als die Reaktionstemperatur liegen. Hierzu können gewöhnliche Autoklaven eingesetzt werden. Zur Beschleunigung der Reaktion wird ein saurer Katalysator zugesetzt. Hierfür eignen sich Brønsted-Säuren wie beispielsweise Salzsäure, Schwefelsäure, Phosphorsäure oder Ammoniumchlorid oder auch saure Aluminiumsilikate oder saure Metalloxide wie beispielsweise Zinn(IV) oxid oder Antimon(III)oxid.

Das Reaktionsgemisch wird erfindungsgemäss ohne Isolierung eines Zwischenprodukts direkt destilliert. Aus praktischen Gründen wird es hierzu typischerweise aus dem Autoklaven in eine Destillationsapparatur übergeführt. Falls eine entsprechend ausgerüstete Apparatur, die sich sowohl für Überdruck als auch für Vakuum eignet, zur Verfügung steht, können selbstverständlich die Umsetzung des Lactons mit dem Amin und die Destillation in ein und derselben Apparatur durchgeführt werden. Bei der Destillation geht zunächst das beim Ringschluss entstandene Wasser und das überschüssige Amin über, danach das Amidin. Je nach Siedepunkt des Produkts wird die Destillation bei entsprechend vermindertem Druck durchgeführt.

Nach dem erfindungsgemässen Verfahren können nicht nur die in der Einleitung erwähnten bekannten bicyclischen Amidine wie beispielsweise DBN und DBU hergestellt werden, sondern insbesondere auch neue Verbindungen aus dieser Stoffklasse mit bisher unerreichten Eigenschaften.

Es wurde gefunden, dass solche bicyclischen Amidine (I), in denen wenigstens einer der Substituenten R¹ bis R¹⁸ eine primäre oder sekundäre Aminogruppe, eine Hydroxygruppe und / oder eine Mercaptogruppe ist bzw. trägt, als Katalysator für die Herstellung von Polyurethanen verwendet werden können und so fest im Polymer gebunden werden, dass weder im Gebrauch noch bei den üblichen Extraktionstests eine Migration feststellbar ist. Vermutlich reagieren diese zusätzlichen funktionellen Gruppen bei der Herstellung des Polyurethans mit den Isocyanatgruppen der Isocyanatkomponente des Polyurethans unter Ausbildung kovalenter Bindungen.

Besonders bevorzugt ist die Herstellung der Verbindungen
3-Amino-2,3,4,6,7,8-hexahydropyrrolo[1,2-a]pyrimidin 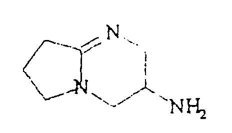
3-(Aminomethyl)-2,5,6,7-tetrahydro-3*H*-pyrrolo[1,2-a]imidazol 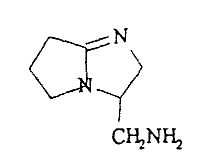
sowohl einzeln als auch als Gemisch
und 3-(Aminomethyl)-3-methyl-2,3,4,6,7,8-hexahydropyrrolo[1,2-a]pyrimidin

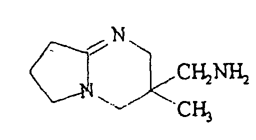

Die nachfolgenden Beispiele verdeutlichen die Durchführung des erfindungsgemässen Verfahrens.

### Beispiel 1

### Herstellung von 2,5,6,7-Tetrahydro-3H-pyrrolo[1,2-a]imidazol (1,4-Diazabicyclo-[3.3.0]oct-4-en)

Ein Gemisch von 129 g (1,5 mol) γ-Butyrolacton, 8 g (0,15 mol) Ammoniumchlorid und 360 g (6 mol) 1,2-Diaminoethan wurde in einem Autoklaven auf 250°C erhitzt. Nach 2,5 h wurde das Gemisch abgekühlt und das überschüssige Diaminoethan und das entstandene Wasser wurden abdestilliert. Der Rückstand wurde bei 200°C / 200 mbar destilliert. Durch Erwärmen des Destillats im Vakuum wurde das restliche Wasser entfernt. Ausbeute: 125 g (76%) farbloses Öl, das allmählich zu einer wachsartigen Masse erstarrt.

### Beispiel 2

### Herstellunq von 3-Amino-2,3,4,6,7,8-hexahydropyrrolo[1,2-a]pyrimidin und 3-(Aminomethyl)-2,5,6,7-tetrahydro-3Hpyrrolo[1,2-a]imidazol

Analog zu Beispiel 1 wurden 21,52 g (0,25 mol) γ-Butyrolacton, 1,34 g (25 mmol) Ammoniumchlorid und 83,43 g (0,94 mol) 1,2,3-Triaminopropan umgesetzt. Der nach Abdestillieren des überschüssigen Amins und des Wassers verbleibende Rückstand wurde bei 170 - 200°C / 0,1 mbar (Badtemperatur) destilliert. Ausbeute: 22,2 g (64%) gelbliches Öl
Kp: 95 - 97°C / 2 mbar

Das Produkt bestand nach GC zu ca. 90% aus dem Isomeren mit Pyrrolo[1,2-a]pyrimidin-Gerüst und zu ca. 10% aus dem Isomeren mit Pyrrolo[1,2-a]imidazol-Gerüst.
3-Amino-2,3,4,6,7,8-hexahydropyrrolo[1,2-a]pyrimidin

| | |
|---|---|
| ¹H-NMR (CDCl₃, 400 MHz) δ: | 1,96 (quint, 2H, H-7); |
| | 2,45 (t, 2H, H-8); |
| | 2,7 - 2,8 (m, 1H, H-4ₐ); |
| | 3,05 (t, 1H, H-2ₐ); |
| | 3,1 - 3,2 (m, 1H, H-3); |
| | 3,3 (t, 2H, H-6); |
| | 3,35 (t, 1H, H-4_{b}); |
| | 3,45 - 3,5 (m, 1H, H-2_{b}). |

| | |
|---|---|
| ¹³C-NMR (100 MHz) δ: | 160,5 (s, C-8a); |
| | 52,88 (t, C-2); |
| | 51,4 (t, C-6); |
| | 50,9 (t, C-4); |
| | 43,0 (t, C-3); |
| | 30,9 (t, C-8); |
| | 20,0 (t, C-7). |

3-(Aminomethyl)-2,5,6,7-tetrahydro-3*H*-pyrrolo[1,2-a]imidazol

| | |
|---|---|
| ¹H-NMR (CDCl₃, 400 MHz) δ: | 2,28 - 2,32 (m, 2H, H-6); |
| | 2,32 - 2,35 (m, 2H, H-7); |
| | 2,75 (m, 1H, CHₐNH₂); |
| | 2,85 (m, 2H, H-5); |
| | 3,3 (m, 1H, CH_{b}NH₂); |
| | 3,4 (m, 1H, H-3); |
| | 3,8 (dd, 1H, H-2ₐ); |
| | 4,15 (dd, 1H, H-2_{b}). |

| | |
|---|---|
| ¹³C-NMR (CDCl₃, 100 MHz) δ: | 175,8 (s, C-7a); |
| | 64,5 (t, C-2); |
| | 64,3 (d, C-3); |
| | 45,8 (t, CH₂NH₂); |
| | 44,3 (t, C-5); |
| | 25,4 (t, C-7); |
| | 22,6 (t, C-6). |

### Beispiel 3

### Herstellung von 3-(Aminomethyl)-3-methyl-2,3,4,6,7,8-hexahydropyrrolo[1,2-a]pyrimidin

Ein Gemisch von 21,03 g (0,24 mol) γ-Butyrolacton und 1,31 g (24 mmol) Ammoniumchlorid in 114,5 g (0,98 mol) 1,1,1-Tris(aminomethyl)ethan wurde in einem Autoklaven auf 250°C erhitzt. Nach 1,5 h wurde das Gemisch abgekühlt und das überschüssige Amin und das entstandene Wasser abdestilliert. Der Rückstand wurde bei 200 - 240°C / 18 mbar destilliert.
Ausbeute: 22,0 g (54%) gelbliches Öl
Kp: 117- 120°C/ 1 mbar

| | |
|---|---|
| ¹H-NMR (CDCl₃, 400 MHz) δ: | 0,91 (s, 3H, CH₃); |
| | 1,95 (quint, 2H, H-7); |
| | 2,45 (t, 2H, H-8); |
| | 2,53 (dd, 2H, CH₂NH₂); |
| | 2,84 (dd, 1H, H-4ₐ); |
| | 3,0 (m, 2H, H-2ₐ, H-4_{b}); |
| | 3,13 (dd, 1H, H-2_{b}); |
| | 3,28 (dt, 2H, H-6). |

| | |
|---|---|
| ¹³C-NMR (CDCl₃, 100 MHz) δ: | 160,1 (s, C-8a); |
| | 53,5 (t, C-2); |
| | 51,5 (t, C-6); |
| | 51,3 (t, C-4) |
| | 48,5 (t, CH₂NH₂); |
| | 32,0 (s, C-3); |
| | 30,9 (t, C-8); |
| | 20,9 (q, CH₃); |
| | 19,9 (t, C-7). |

### Beispiel 4

### Herstellung von 2,5,6,7-Tetrahydro-3H-pyrrolo[1,2-a]imidazol

Ein Gemisch von 86 g (1 mol) γ-Butyrolacton und 2 ml 98%iger Schwefelsäure in 240 g (4 mol) 1,2-Diaminoethan wurde in einem Autoklaven auf 250°C erhitzt. Nach 4,5 h wurde das Reaktionsgemisch abgekühlt und wie in Beispiel 1 beschrieben aufgearbeitet.
Ausbeute: 90 g (82%), Eigenschaften siehe Beispiel 1.

### Beispiel 5

### Herstellung von 2,3,4,6,7,8-Hexahydropyrrolo[1,2-a]pyrimidin (1,5-Diazabicyclo-[4.3.0]non-5-en, DBN)

Ein Gemisch von 43 g (0,5 mol) γ-Butyrolacton und 2,68 g (25 mmol) Ammoniumchlorid in 148 g (2 mol) 1,3-Diaminopropan wurde in einem Autoklaven auf 250°C erhitzt. Nach 4,5 h wurde das Gemisch abgekühlt und das überschüssige Amin und das gebildete Wasser bis 250 mbar abdestilliert. Der Rückstand wurde im Vakuum destilliert.
Ausbeute: 47 g (75%) farblose Flüssigkeit
Kp: 98°C / 12 mbar

### Beispiel 6

### Herstellung von 2,3,4,6,7,8,9,10-Octahydropyrimido[1,2-a]azepin (1,8-Diazabicyclo-[5.4.0]undec-7-en, DBU)

Es wurde verfahren wie in Beispiel 5 beschrieben, mit dem Unterschied, dass anstelle von γ-Butyrolacton 57 g (0,5 mol) ε-Caprolacton eingesetzt wurden.
Ausbeute: 16 g (21%) farblose Flüssigkeit
Kp: 115°C / 8 mbar

## Patentansprüche

1. Verfahren zur Herstellung' bicyclischer Amidine der allgemeinen Formel 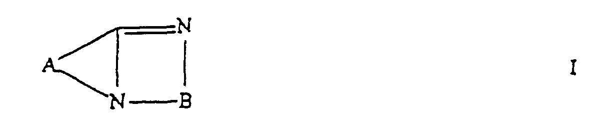 worin A ausgewählt ist aus der Gruppe -CR¹R²-CR³R⁴-CR⁵R⁶-, -CR¹R²-CR³R⁴-CR⁵R⁶-CR⁷R⁸-, -CR¹R²-CR³R⁴-CR⁵R⁶-CR⁷R⁸-CR⁹R¹⁰-, worin die Substituenten in A jeweils vom Stickstoffatom ausgehend numeriert sind und B ausgewählt ist aus der Gruppe -CR¹¹R¹²-CR¹³R¹⁴-, -CR¹¹R¹²-CR¹⁵R¹⁶-CR¹³R¹⁴-, -CR¹¹R¹²-CR¹⁵R¹⁶-CR¹⁷R¹⁸-CR¹³R¹⁴- und R¹, R² und R¹¹ bis R¹⁴ jeweils unabhängig voneinander Wasserstoff, C₁-C₄-Alkyl, Phenyl, alkylsubstituiertes Phenyl oder mit Hydroxy, Amino, C₁-C₄-Alkylamino oder Mercapto substituiertes C₁-C₄-Alkyl und R³ bis R¹⁰ und R¹⁵ bis R¹⁸ jeweils unabhängig voneinander Wasserstoff, C₁-C₄-Alkyl, Phenyl, alkylsubstituiertes Phenyl, Hydroxy, Amino, C₁-C₄-Alkylamino, Mercapto oder mit Hydroxy, Amino, C₁-C₄-Alkylamino oder Mercapto substituiertes C₁-C₄-Alkyl bedeuten, **dadurch gekennzeichnet, dass** ein Lacton der allgemeinen Formel 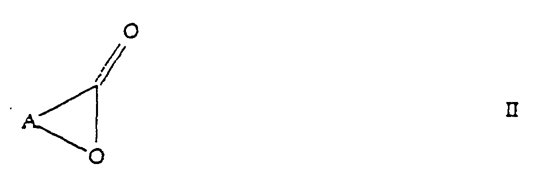 worin A die oben genannte Bedeutung hat, mit einem Amin der allgemeinen Formel
H₂N―B―NH₂ III
worin B die oben genannte Bedeutung hat, in Gegenwart eines sauren Katalysators auf wenigstens 150°C erhitzt wird wobei das Amin (III) in einer Menge von 2 bis 20 mol auf I mol des Lactons (II) eingesetzt wird, und das so erhaltene Reaktionsgemisch ohne Isolierung eines Zwischenprodukts einer fraktionierten Destillation unterworfen wird.

2. Verfahren nach Anspruch 1, **dadurch gekennzeichnet, dass** als Lacton (II) eine Verbindung aus der Gruppe γ-Butyrolacton, γ-Valerolacton, δ-Valerolacton, ε-Caprolacton, Pantolacton eingesetzt wird.

3. Verfahren nach Anspruch 1 oder 2, **dadurch gekennzeichnet, dass** als Amin (III) eine Verbindung aus der Gruppe 1,2-Diaminoethan, 1,2-Diaminopropan, 1,3-Diaminopropan, 1,2,3-Triaminopropan, 1,1,1-Tris(aminomethyl)ethan, Tetrakis(aminomethyl)methan eingesetzt Wird.

## Claims

1. Process for the preparation of bicyclic amidines of the general formula 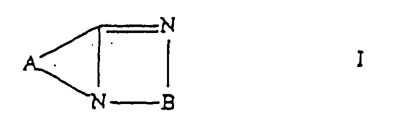 wherein A is chosen from the group consisting of -CR¹R²-CR³R⁴-CR⁵R⁶-, -CR¹R²-CR³R⁴-CR⁵R⁶-CR⁷R⁸- and CR¹R²-CR³R⁴-CR⁵R⁶-CR⁷R⁸-CR⁹R¹⁰-, wherein the substituents in A are in each case numbered starting from the nitrogen atom
and B is chosen from the group consisting of -CR¹¹R¹²-CR¹³R¹⁴-, -CR¹¹R¹²-CR¹⁵R¹⁶-CR¹³R¹⁴- and -CR¹¹R¹²-CR¹⁵-R¹⁶-CR¹⁷R¹⁸-CR¹³R¹⁴ - and R¹, R² and R¹¹ to R¹⁴ in each case independently of one another denote hydrogen, C₁-C₄-alkyl, phenyl, alkyl-substituted phenyl or C₁-C₄-alkyl which is substituted by hydroxyl, amino, C₁-C₄-alkylamino or mercapto and R³ to R¹⁰ and R¹⁵ to R¹⁸ in each case independently of one another denote hydrogen, C₁-C₄-alkyl, phenyl, alkyl-substituted phenyl, hydroxyl, amino, C₁-C₄-alkylamino, mercapto or C₁-C₄-alkyl which is substituted by hydroxyl, amino, C₁-C₄-alkylamino or mercapto, **characterized in that** a lactone of the general formula 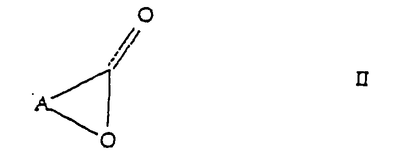 wherein A has the abovementioned meaning, is heated to at least 150°C with an amine of the general formula
H₂N-B-NH₂ III
wherein B has the abovementioned meaning, in the presence of an acid catalyst, the amine (III) being employed in an amount of 2 to 20 mol per 1 mol of the lactone (II), and the reaction mixture obtained in this way is subjected to fractional distillation without isolation of an intermediate product.

2. Process according to claim 1, **characterized in that** a compound from the group consisting of γ-butyrolactone, γ-valerolactone, δ-valerolactone, ε-caprolactone and pantolactone is employed as the lactone (II).

3. Process according to claim 1 or 2, **characterized in that** a compound from the group consisting of 1,2-diaminoethane, 1,2-diaminopropane, 1,3-diaminopropane, 1,2,3-triaminopropane, 1,1,1-tris(aminomethyl)ethane and tetrakis(aminomethyl)methane is employed as the amine (III).

## Revendications

1. Procédé pour la préparation d'amidines bicycliques de la formule générale 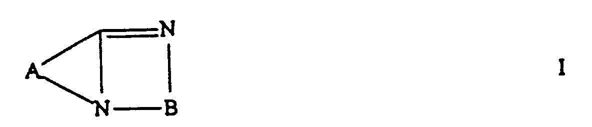 dans laquelle A est choisi dans le groupe -CR¹R²-CR³R⁴-CR⁵R⁶-, -CR¹R²-CR³R⁴-CR⁵R⁶-CR⁷R⁸-, -CR¹R²-CR³R⁴-CR⁵R⁶-CR⁷R⁸-CR⁹R¹⁰-, où les substituants sont numérotés dans A respectivement en partant de l'atome d'azote
et B est choisi dans le groupe
-CR¹¹R¹²-CR¹³R¹⁴-, -CR¹¹R¹²-CR¹⁵R¹⁶-CR¹³R¹⁴-, -CR¹¹R¹²-CR¹⁵R¹⁶-CR¹⁷R¹⁸-CR¹³R¹⁴
et R¹, R² et R¹¹ jusqu'à R¹⁴ signifient respectivement, indépendamment l'un de l'autre, l'hydrogène, alkyle C₁-C₄, phényle, phényle substitué par alkyle ou par hydroxy, amino, alkylamino C₁-C₄, ou alkyle C₁-C₄ substitué par mercapto, et R³ jusqu'à R¹⁰ et R¹⁵ jusqu'à R¹⁸ signifient respectivement, indépendamment l'un de l'autre, l'hydrogène, alkyle C₁-C₄, phényle, phényle substitué par alkyle, hydroxy, amino, alkylamino C₁-C₄, mercapto ou alkyle C₁-C₄ substitué par hydroxy, amino, alkylamino C₁-C₄ ou par mercapto, **caractérisé en ce qu'**une lactone de la formule générale 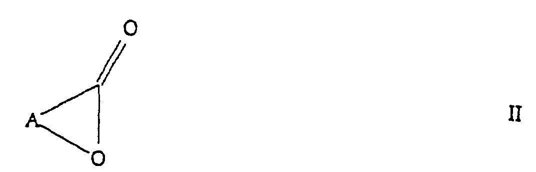 dans laquelle A a la signification ci-dessus, avec une amine de la formule générale
H₂N―B―NH₂ III
dans laquelle B a la signification ci-dessus en présence d'un catalyseur acide, est chauffée jusqu'à au moins 150°C et l'amine (III) est incorporée dans une quantité de 2 à 20 moles pour 1 mole de lactone (II), de sorte que le mélange réactionnel ainsi obtenu est soumis sans isolation d'un produit intermédiaire à une distillation fractionnée.

2. Procédé selon la revendication 1, **caractérisé en ce qu'**en tant que lactone (II), on incorpore une composition à partir du groupe γ-butyrolactone, γ-valérolactone, δ-valérolactone, ε-caprolactone, pantolactone.

3. Procédé selon la revendication 1 ou 2, **caractérisé en ce qu'**en tant qu'amine (III), on utilise une composition de groupe 1,2-diaminoéthane, 1,2-diaminopropane, 1,3-diaminopropane, 1,2,3-triaminopropane, 1,1,1-tris(aminométhyl)éthane, tétrakis(aminométhyl)méthane.
